# EUROPEAN PATENT APPLICATION

(11) **EP 2 319 913 A1**
(43) Date of publication of application: **11.05.2011**
(21) Application number: 08876566.4
(22) Date of filing: 18.07.2008
(51) Int. Cl.: C12M 1/42, C12M 3/00, C12N 15/87, C12Q 1/00

(54) **A MULTIPURPOSE MICRO ELECTRIC FIELD NETWORKING CELL PROCESSING DEVICE**

(71) Applicant: Suntek Medical Scientific And Technologies (Shanghai), Pudong New Area, Shanghai 201201 (CN)
(72) Inventor: SEN, Luyi, Shanghai 201201 (CN)
(74) Representative: Brown, David Leslie
(86) International application number: PCT/CN2008/071685
(87) International publication number: WO 2010/006482

(57) **Abstract**

A multipurpose micro electric field network cell processing device comprises: a hollow frame structure; transparent film layers which are respectively correspondingly arranged on the upper side surface and the lower side surface of the frame; electrode groups which are arranged on the transparent film layer on at least one side and used for generating a low strength electric field; wire connecting terminals of the electrode groups arranged on at least one end of the frame; and an inlet and an outlet of culture/processing solution arranged at two corresponding ends of the frame, wherein sandwich space surrounded by the two transparent film layers and the frame constitutes a cell culture/processing chamber. The device can effectively introduce or deliver a drug into target cells under a low strength electric field network environment, monitor and control the whole experiment/processing process more precisely and be conducive to separation and selection of the target cells.

## Description

### Technical Field

The invention belongs to the field of microbiology, in particular to a microbiology device for cell processing.

### Background of the Invention

In biological experiments, culture of bacteria or cells and various medical research processes, a bacteria/cell culture dish (also known as Petri dish) is an essential test vessel or a cell processing device.

Generally, the bacterial/cell culture dish is made of a transparent material and is a disk-like or dish-like container with a raised rim, and culture medium or culture solution is contained therein; culture targets (bacteria or cells) are arranged on the culture medium or in the culture solution, and the culture dish is usually equipped with a cover for closing or sealing, thereby preventing the targets from being contaminated.

In the experimental process, the culture dish carrying the culture medium or the culture solution and the culture targets is generally placed at a certain temperature for carrying out culture or preservation of the bacteria or the cells.

Due to the universality of using the culture dish in the biological industry, the medical industry or the light industry, the efforts to improve the structure of the culture dish have never been stopped. The Chinese patent application of Petri dish with dual-side use with the publication date of October 8, 2003 and the publication No. of CN 1446903A is the one of such efforts.

Along with the change in experimental means and the development of the biomedical technologies, an external acting electric field often needs to be added on the culture targets for obtaining the growth results or the change trends of the bacteria or the cells under certain specific conditions.

The existing culture dish which can impose the acting electric field during the culture process usually adopts the structural form of using two external electrode needles, such as the Chinese patent cell culture dish with the publication date of August 1, 2001 and the publication No. of CN 2440820, wherein a groove-shaped cell culture chamber is formed at the center, the height being 2.0mm at least, and the outer diameter being matched with a groove on a bracket. An upper cover with a hole at the center is arranged on the cell culture dish, two electrodes can be vertically inserted on the upper cover, and the inserting ends of the electrodes are mutually bent into the horizontal direction in parallel and positioned at the bottom part of the cell culture chamber.

Although the technical scheme solves the problem of introducing the acting electric field during the cell culture and processing process, the acting electric field produced in the culture dish has non-uniform strength and is inconvenient to observe experimental results under a microscope and other inconveniences due to the limited structural form of the electrodes.

The inventor of the application provides and invents a method for effectively introducing or delivering drugs, genes, siRNA (small interfering RNA), shRNA (short hairpin RNA), proteins, peptides, antibodies or other biomedical and therapeutic molecules and preparations into cells under the condition of an external weak electric field network through many years of studies and practices, which comprises the steps of adopting a network formed by an electrode array, using a low-voltage electric field to impose short-term pulses and/or a pulse group for a long time, carrying out electroosmosis on cell membranes, and further conveying the genes, the proteins and the drugs into in vitro and in vivo skin, soft tissues and bones (in cells) in a targeting manner, and the specific contents can refer to the international patent application of method for delivering genes, proteins and drugs into in vitro cells under mediation of ultra-low strength electric field network with the international application No. of PCT/US2006/011355 and the application date of March 16, 2006 and the internal patent application of method and device for delivering drugs, genes, siRNA, shRNA, proteins, peptides, antibodies or other biomedical and therapeutic molecules and preparations into skin, soft tissues, joints and bones under mediation of low strength electric field network with the international application No. of PCT/US2007/008445 and the application date of April 2, 2007, as well as the related contents in the Chinese patent application biological and medical multi-channel low-voltage micro electric field generator with the application No. of 200810036767.4 and the PCT application biological and medical multi-channel low-voltage micro electric field generator with the application No. of PCT/CN/2008/001022. We should declare that the contents disclosed in the reference documents shall be considered as background information or explanatory information of this application rather than certain limitation or definition of this application.

The nature of the mode of an electromagnetic field provided by the network in the above-mentioned reference documents is different from the nature of the mode of the electromagnetic field provided by the conventional electroporation technology; the electromagnetic field is not the electromagnetic field of electroporation, but is the low strength electric field network (LSEFN).

The inventor of the application believes that the bioelectric mechanism and the nature used in the invention are different from those of the existing electroporation technology, thereby being misled and incorrect to call the bioelectric application of the invention as the electroporation. Therefore, the bioelectric application of the invention is called as the low strength electric field network (LSEFN) in the following text of the application and the medical field.

The low strength electric field network (LSEFN) can be used for carrying out transfer of drugs and biomass in cells for the in vitro cells and the tissues. The bioengineered cells and the tissues can be further systematically input, delivered or implanted into various organs or tissues for treating diseases.

The low strength electric field network (LSEFN) can also be used for carrying out targeting transfer of the drugs and the biomass in the cells for the in vivo organs and the tissues.

Now, there is no culture dish for imposing the acting electric field on the culture target cells under the condition of the low strength electric field network or a cell processing device which can impose the acting electric field on the culture target cells under the condition of meeting the low strength electric field network.

### Summary of the Invention

The technical problem to be solved by the invention is to provide a multipurpose micro electric field network cell processing device which not only has the conventional functions of the existing cell processing device, but also provides a low strength electric field network environmental condition for target cells, thereby being convenient to carry out drug transfer for the target cells, or effectively introducing or delivering drugs, genes, siRNA, shRNA, proteins, peptides, antibodies or other biomedical and therapeutic molecules and preparations into cells, realizing flowability, replaceability and mensurability of cell culture/processing solution and monitoring and controlling the whole experimental/processing process more precisely through various measurement data.

The technical scheme of the invention is as follows: the invention provides a multipurpose micro electric field networking cell processing device which comprises a hallow frame structure, wherein two transparent film layers are respectively and correspondingly arranged on the upper side surface and lower side surface of the frame; electrode groups are provided on the transparent film layer of at least one side of the frame for generating a low strength electric field; wire connecting terminals of the electrode groups are arranged on at least one end of the frame; an inlet and an outlet of culture-processing solution are arranged at two corresponding ends of the frame; a sandwich space surrounded by the two transparent film layers and the frame constitutes a cell culture-processing chamber.

Wherein the framework is in the shape of a rectangle or a regular polygon, bearing edges are arranged on the upper side surface and the lower side surface of an inner edge of the framework, and the transparent film layers are bondedly/fixedly connected with the framework into a whole via the bearing edges.

The transparent film layers are transparent plastic thin film layers.

The electrode groups are printed circuit copper foil layers which are distributed in the transparent film layer or on the surface of the transparent film layer by a structure of leading positive electrodes and negative electrodes to be arranged alternately, wherein the positive and the negative electrode groups are electrically connected with the wire connecting terminals positioned at one end of the framework and an external instrument via omnibus bars.

The electrode groups are arranged in the transparent film layer, pass through platinum/tungsten points which are connected with the electrode groups and penetrate the surface of the transparent film and further constitute an electrode lattice group on the surface of the transparent film.

Or the positive and the negative electrode groups can be arranged in the same transparent film layer, or be arranged on two sides of the same transparent film layer, or be respectively correspondingly arranged in the two corresponding transparent film layers, or be respectively correspondingly arranged on one surface of the two corresponding transparent film layers.

The inlet and the outlet of the culture/processing solution are arranged in a framework body, one end of each is in a converging cone-like opening structure, and the other end of each is communicated with the cell culture/processing chamber.

The cell culture/processing chamber is a thin-layer empty chamber, and the thickness is not more than 2mm.

Further, a scale/graduated scale is arranged on the periphery of the framework.

Compared with the prior art, the invention has the following advantages:
1. The invention creates conditions for establishing a low strength electric field network , environment for the cell culture/processing chamber by setting the positive and the negative electrode groups, thereby realizing the purpose of the invention of effectively introducing or delivering the drugs, the genes, the siRNA, the shRNA, the proteins, the peptides, the antibodies or other biomedical and therapeutic molecules and the preparations into the target cells under the environmental condition of the low strength electric field network.
2. As the inlet and the outlet of the culture/processing solution are arranged, the invention can realize the flowability, the replaceability and the mensurability of the cell culture/processing solution, in addition to the conventional functions of the existing cell culture/processing device, the inlet and the outlet of the culture/processing solution can also be connected with various biological, physical and chemical meters, thereby monitoring and controlling the whole experimental/processing process more precisely through the various measurement data.
3. The adoption of the cuttable structure of the transparent film layers can facilitate the separation and the selection of the culture/processing target cells and be convenient to be observed under a microscope as a whole.

### Brief Description of the Drawings

Figure 1 is a schematic diagram of structure of framework;
Figure 2 is an A-A' section view of framework in Figure 1.
Figure 3 is a B-B' section view of framework in Figure 1.
Figure 4 is a schematic diagram of structure of electrode groups in transparent thin film
Figure 5 is an amplified schematic diagram of local structure of C part in Figure 4.

In the figures, 1 refers to framework, 2 refers to bearing edges, 3-1 and 3-2 refer to inlet and outlet of culture/processing solution, 4-1 and 4-2 are wire connecting terminals of electrode groups, 5, 5-1 and 5-2 refer to transparent film layers, 6 refers to scale/graduated scale, 7 refers to cell culture/processing chamber, 8 and 9 refer to omnibus bars of positive and negative electrodes, 10 refers to platinum/tungsten points, and 11 and 12 refer to positive and negative electrodes.

### Detailed Description of the Invention

In combination with figures, the invention is further described as follows:

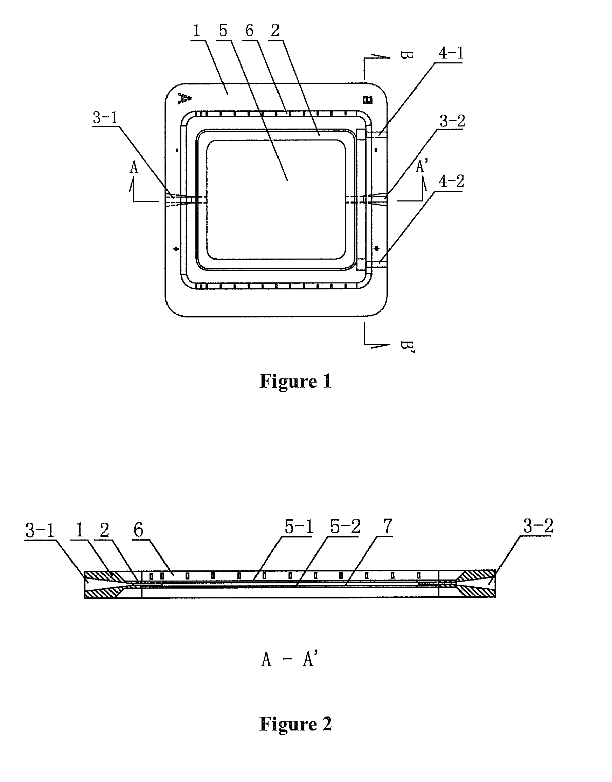

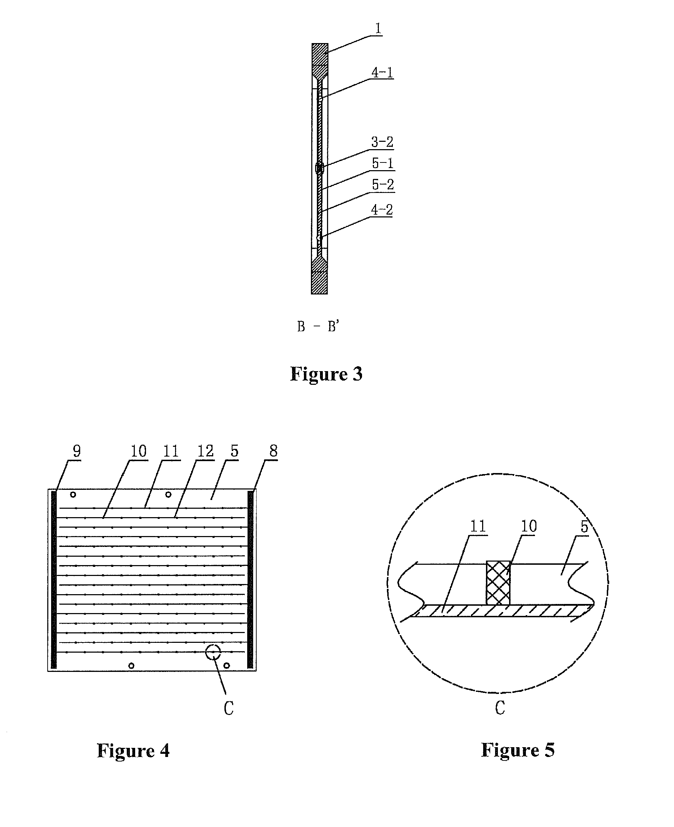

In Figure 1, the processing device is provided with a hollow framework structure 1, transparent film layers 5 are respectively correspondingly arranged on the upper side surface and the lower side surface of the framework, electrode groups (which are not shown in the figure for simplicity, and the specific is shown in Figure 4) for producing a low strength electric field are arranged on the transparent film layer on at least one side, wire connecting terminals 4-1 and 4-2 of the electrode groups are arranged at one end of the framework, an inlet and an outlet 3-1 and 3-2 of culture/processing solution are correspondingly arranged at two corresponding ends of the framework, and sandwich space surrounded by the two transparent film layers and the framework constitutes a cell culture/processing chamber.

Wherein the framework is in the shape of a rectangle or a regular polygon, bearing edges 2 are arranged on the upper side surface and the lower side surface of an inner edge of the framework, and the transparent film layers are bonded/fixedly connected with the framework into a whole via the bearing edges.

The transparent film layers are transparent plastic thin film layers.

In Figure 2, the inlet and the outlet 3-1 and 3-2 of the culture/processing solution are arranged in a framework 1 body, one end of each is in a converging cone-like opening structure, and the other end of each is communicated with the cell culture/processing chamber 7.

The sandwich space surrounded by the two transparent film layers 5-1 and 5-2 and the framework constitutes the cell culture/processing chamber 7.

It can be seen from the figure that the cell culture/processing chamber is a thin-layer chamber, and the thickness is not more than 2mm.

Further, a scale/graduated scale 6 is arranged on the periphery of the framework.

In Figure 3, the wire connecting terminals 4-1 and 4-2 of the electrode groups are arranged at one end of the framework 1 and respectively in contact and connection with omnibus bars 8 and 9 of positive and negative electrodes (shown in Figure 4) arranged on the transparent film layer for forming electrical pathways.

The inlet and the outlet of the culture/processing solution are arranged at two corresponding ends of the framework, and the outlet 3-2 of the culture/processing solution can be just seen in the section view direction shown in the figure.

In Figure 4, the electrode groups are printed circuit copper foil layers which are distributed in the transparent film layer 5 or on the surface of the transparent film layer by a structure of leading positive electrodes and negative electrodes 11 and 12 to be arranged alternately, wherein the positive and the negative electrode groups are electrically connected with the wire connecting terminals (see 4-1 and 4-2 in Figure 4) positioned at one end of the framework and an external instrument via the omnibus bars 8 and 9.

The electrode groups 11 and 12 are arranged in the transparent film layer, pass through platinum/tungsten points 10 which are connected with the electrode groups and penetrate the surface of the transparent film and further constitute an electrode lattice group on the surface of the transparent film.

The distribution structure of the positive and the negative electrodes shown in the figure is only the schematic diagram of the structure, when in practical implementation, the positive and the negative electrodes are not only limited to the distribution form shown in the figure, and can adopt the way of arranging them alternately in a two-and-two way or other forms, in order to obtain the better electric field distribution effect.

In addition, the printed circuit copper foil layers constituting the electrode groups can be arranged in the same transparent film layer, or be arranged on two corresponding side surfaces of the same transparent film layer, or be respectively correspondingly arranged in two corresponding transparent film layers, or be respectively correspondingly arranged on certain surfaces of the two corresponding transparent film layers, aiming at obtaining the better electric field distribution effect or facilitating the processing and the manufacturing.

In Figure 5, the printed circuit copper foil layers 11 are arranged on one side surface of the transparent film layer, pass through platinum/tungsten points 10 which are connected therewith and penetrate the surface of the transparent film 5 and further constitute the electrode lattice group on the surface of the transparent film.

Due to the adoption of the structural form of the electrode lattice group, compared with the prior art, the electric field distribution of the produced low strength electric field network is more uniform, and the applied voltage can be as low as millivolt level, thereby being safer for target cells and avoiding that the long-time imposing of the acting electric field causes the electrical breakdown injuries to the target cells.

The implementation of the invention is conductive to realizing the purposes of effectively introducing or delivering drugs, genes, siRNA, shRNA, proteins, peptides, antibodies or other biomedical and therapeutic molecules and preparations into the target cells under the environmental condition of the low strength electric field network; simultaneously, in addition to having the conventional functions of the existing cell culture/processing device, the invention can further monitor and control the whole experimental/processing process, be convenient to be observed under a microscope as a whole and be conductive to separation and selection of the culture/processing target cells.

The above embodiments are only used to explain and describe the invention rather than limiting the technical scheme of the invention; and those of ordinary skill in the art shall recognize that any changes and variations within the real spirit range can be determined to fall within the protection scope of the claimed invention.

The invention can be widely applied in the fields of clinical medicine, basic experiments and bioscience

## Claims

1. A multipurpose micro electric field network cell processing device, comprising a hallow frame structure, wherein two transparent film layers are respectively and correspondingly arranged on the upper side surface and lower side surface of the frame; electrode groups are provided on the transparent film layer of at least one side of the frame for generating a low strength electric field; wire connecting terminals of the electrode groups are arranged on at least one end of the frame; an inlet and an outlet of culture-processing solution are arranged at two corresponding ends of the frame; a sandwich space surrounded by the two transparent film layers and the frame constitutes a cell culture-processing chamber.

2. The multipurpose micro electric field network cell processing device according to claim 1, wherein the frame is in the shape of a rectangle or a regular polygon, bearing edges are arranged on the upper side surface and the lower side surface of an inner edge of the frame, and the transparent film layers are bondedly/fixedly connected with the frame into a whole via the bearing edges.

3. The multipurpose micro electric field network cell processing device according to claim 1, wherein the transparent film layers are transparent plastic thin film layers.

4. The multipurpose micro electric field network cell processing device according to claim 1, wherein that the electrode groups are printed circuit copper foil layers which are distributed in the transparent film layer or on the surface of the transparent film layer by a structure of leading positive electrodes and negative electrodes to be arranged alternately, wherein the positive and the negative electrode groups are electrically connected with the wire connecting terminals positioned at one end of the frame and an external instrument via omnibus bars.

5. The multipurpose micro electric field network cell processing device according to claim 4, wherein the electrode groups are arranged in the transparent film layer, pass through platinum/tungsten points which are connected with the electrode groups and penetrate the surface of the transparent film and further constitute an electrode lattice group on the surface of the transparent film.

6. The multipurpose micro electric field network cell processing device according to claim 4, wherein that the positive and the negative electrode groups can be arranged in the same transparent film layer, or be arranged on two sides of the same transparent film layer, or be respectively correspondingly arranged in the two corresponding transparent film layers, or be respectively correspondingly arranged on one surface of the two corresponding transparent film layers.

7. The multipurpose micro electric field network cell processing device according to claim 1, wherein the inlet and the outlet of the culture/processing solution are arranged in a frame body, one end of each is in a converging cone-like opening structure, and the other end of each is communicated with the cell culture/processing chamber.

8. The multipurpose micro electric field network cell processing device according to claim 1, wherein the cell culture/processing chamber is a thin-layer empty chamber, and the thickness is not more than 2mm.

9. The multipurpose micro electric field network cell processing device according to claim 1, wherein a scale/graduated scale is arranged on the periphery of the frame.
